# EUROPÄISCHE PATENTANMELDUNG

(11) **EP 4 311 824 A1**
(43) Veröffentlichungstag der Anmeldung: **31.01.2024**
(21) Anmeldenummer: 22020358.2
(22) Anmeldetag: 25.07.2022
(51) Int. Cl.: C07C 41/01, C07C 43/04

(54) **VERFAHREN UND ANLAGE ZUR HERSTELLUNG VON DIMETHYLETHER**

(71) Anmelder: Linde GmbH, 82049 Pullach (DE); Technische Universität München, 80333 München (DE)
(72) Erfinder: Klein, Harald, 82049 Pullach (DE); Peschel, Andreas, 82049 Pullach (DE); Hemauer, Johanna, 82049 Pullach (DE); Meier, Carolin, 82049 Pullach (DE)
(74) Vertreter: Fischer, Werner

(57) **Zusammenfassung**

Ein Verfahren (100, 200) zur Herstellung von Dimethylether wird vorgeschlagen, bei dem ein die Reaktanden Kohlenmonoxid und/oder Kohlendioxid und Wasserstoff enthaltendes Synthesegas unter teilweiser Umsetzung der Reaktanden und unter Bildung von Methanol an einem oder mehreren Methanolkatalysatoren und unter Umsetzung zumindest eines Teils des Methanols an einem oder mehreren Dimethyletherkatalysatoren einer Dimethylethersynthese (30) unterworfen wird, wobei der eine oder die mehreren Methanolkatalysatoren und der eine oder die mehreren Dimethyletherkatalysatoren in einem gemeinsamen Katalysatorbett bereitgestellt sind, wobei der Dimethylethersynthese (30) ein Rohprodukt (101) entnommen wird, das neben Dimethylether auch Wasser, Methanol und leichte Gase enthält, wobei die leichten Gase Kohlendioxid, Kohlenmonoxid und Wasserstoff umfassen, und wobei zumindest ein Teil des Rohprodukts (101) einer ersten Trennung unterworfen wird, in der ein gegenüber dem Rohprodukt (101) an Wasser und Methanol angereichertes und an Dimethylether und den leichten Gasen abgereichertes, aber einen Teil der leichten Gase enthaltendes, flüssiges Trennprodukt gebildet wird. Es ist vorgesehen, dass aus zumindest einem Teil des flüssigen Trennprodukts unter Verwendung keiner oder höchstens einer zweiten Trennung ein oder mehrere Rückführströme (102, 104, 126) gebildet wird oder werden, dass von dem einen oder den mehreren Rückführströmen ein oder jeweils ein Teil (124, 131) aus dem Verfahren ausgeführt wird, und dass ein oder jeweils ein verbleibender Rest hiervon zumindest zum Teil oder jeweils zumindest zum Teil zu der Dimethylethersynthese (30) zurückgeführt wird. Eine entsprechende Anlage ist ebenfalls Gegenstand der vorliegenden Erfindung.

## Beschreibung

Die Erfindung betrifft ein Verfahren und eine Anlage zur Herstellung von Dimethylether.

### Hintergrund

Bei Dimethylether (DME) handelt es sich um den strukturell einfachsten Ether. Dimethylether enthält zwei Methylgruppen als organische Reste. Dimethylether ist polar und findet herkömmlicherweise in flüssiger Form als Lösungsmittel Verwendung. Dimethylether kann ferner als Kühlmittel eingesetzt werden und herkömmliche Fluorchlorkohlenwasserstoffe ersetzen. Neuerdings wird Dimethylether zunehmend als Ersatz für Brenngas (Flüssiggas) und Kraftstoffe wie Diesel eingesetzt.

Dimethylether kann aus Erd- oder Biogas über die Zwischenstufen Synthesegas und Methanol erzeugt werden. Hierbei wird das Erd- oder Biogas zunächst zu Synthesegas umgesetzt. Das Synthesegas wird anschließend in einer zweistufigen Reaktion über Methanol zu Dimethylether konvertiert.

Unter Synthesegas soll hier ein Gasgemisch verstanden werden, das zumindest Kohlenmonoxid und Wasserstoff in wechselnden Anteilen, die zusammen jedoch den überwiegenden Teil des Gasgemischs ausmachen, enthält. Auch Kohlendioxid kann enthalten sein, welches durch eine reverse Wassergasshiftreaktion (RWGS) zu weiterem Kohlenmonoxid umgesetzt werden kann.

Synthesegas kann durch Pyrolyse von Kohle, Öl, kohlenstoffhaltigen Abfällen, Biomasse oder anderen Ausgangsstoffen, durch Trockenreformierung (Dry Reforming) von Erdgas mit Kohlendioxid, durch Dampfreformierung (Steam Reforming) von Erdgas, durch autotherme Reformierung (ATR) von Erdgas, durch partielle Oxidation (POX) von Kohlenwasserstoffen, insbesondere Erdgas bzw. Methan, oder Kombinationen aus den genannten Verfahren, gewonnen werden.

Die Synthese von Dimethylether aus Synthesegas ist thermodynamisch und wirtschaftlich vorteilhaft gegenüber einer Synthese aus Methanol.

Die vorliegende Erfindung betrifft insbesondere die sogenannte direkte Synthese von Dimethylether, worunter eine Synthese verstanden wird, bei der sämtliche Reaktionen in ein und demselben Reaktor und in ein und demselben Katalysatorbett ablaufen. Bei den ablaufenden Reaktionen handelt es sich um eine Reaktion von Wasserstoff mit Kohlenmonoxid und/oder Kohlendioxid zu Methanol und eine (Weiter-) Reaktion von Methanol zu Dimethylether und Wasser. Die direkte Synthese von Dimethylether ist beispielsweise aus der US 4,536,485 A und der US 5,189,203 A bekannt. Herkömmlicherweise werden hierbei Hybridkatalysatoren verwendet. Die Reaktion ist exotherm und erfolgt typischerweise bei einer Temperatur von 200 bis 300 °C und bei einem Druck von 20 bis 100 bar.

Zur direkten Synthese von Dimethylether werden normalerweise aufrechtstehende Rohrreaktoren eingesetzt, die von unten mit druckbeaufschlagtem und erhitztem Synthesegas beschickt werden. Ein aus dem Rohrreaktor erhaltener Produktstrom wird kopfseitig entnommen, gekühlt und einer Trennung zugeführt.

Der Produktstrom enthält typischerweise neben Dimethylether nicht umgesetzte Komponenten des Synthesegases sowie weitere Reaktionsprodukte. Typischerweise weist der Produktstrom neben Dimethylether zumindest Methanol, Wasser, Kohlendioxid, Kohlenmonoxid und Wasserstoff sowie in geringerer Menge Methan, Ethan, organische Säuren und höhere Alkohole auf.

In einem Gasgemisch, das aus dem Produktstrom gebildet wird, sind damit typischerweise neben Dimethylether noch Kohlendioxid und leichter als Kohlendioxid siedende Komponenten wie Wasserstoff und Kohlenmonoxid enthalten. Je nach Produktspezifikation müssen diese zumindest teilweise abgetrennt werden.

Die Zusammensetzung des Produktstroms (dieser oder ein hieraus gebildetes Gasgemisch wird nachfolgend auch als Dimethylether enthaltendes Produktgemisch bezeichnet) wird dabei insbesondere durch die gewählten Reaktionsbedingungen während der Synthese bestimmt. Je näher diese Zusammensetzung stromab des Reaktors an der geforderten Spezifikation liegt, desto geringer ist der Aufwand, der zur Aufreinigung des Produktstroms betrieben werden muss.

Insbesondere in Bezug auf die hier verwendete Terminologie, aber auch auf grundsätzliche Überlegungen die (insbesondere direkte) Synthese von Dimethylether aus Synthesegas betreffend, sei auf den Artikel von Kiendl et al., Chem. Ing. Tech. 2020, 92, No. 6, 736-745, verwiesen.

In der Praxis weisen sowohl bekannte direkte Reaktionsverfahren zur Herstellung von Dimethylether als auch Verfahren, bei denen Methanol als Zwischenprodukt abgetrennt wird, gewisse Nachteile auf, die die vorliegende Erfindung überwinden will.

### Offenbarung der Erfindung

Diese Aufgabe wird durch ein Verfahren und eine Anlage mit den Merkmalen der jeweiligen unabhängigen Patentansprüche gelöst. Vorteilhafte Ausgestaltungen sind Gegenstand der Unteransprüche sowie der nachfolgenden Beschreibung.

Bei der herkömmlichen Dimethylethersynthese wird, wie erwähnt, in einem ersten Schritt Methanol hergestellt, welches in weiteren Schritt und an einem separaten Katalysatorbett bzw. in einem separaten Reaktor zu Dimethylether konvertiert wird. Beide Reaktionen werden separat durchgeführt und sind gleichgewichtslimitiert. Der Vorteil der Kombination beider Reaktionen in einer direkten Synthese besteht insbesondere im Potential zur Realisierung höherer Umsätze, die bei den herkömmlichen Verfahren nicht genutzt wird.

Je nach Stöchiometriezahl des bei der einstufigen Dimethylethersynthese frisch eingesetzten Synthesegases entstehen entweder (vorwiegend) Dimethylether und Wasser (bei einer Stöchiometriezahl von 2 und mehr) oder es entstehen (vorwiegend) Dimethylether und Kohlendioxid (bei einer Stöchiometriezahl von 1). Dies liegt darin begründet, dass unterhalb einer Stöchiometriezahl von 2 Synthesegas nicht mehr vollständig zu Dimethylether und Wasser umgesetzt werden und zusätzlich Kohlendioxid gebildet wird, dass abgetrennt werden muss. Bei einer Stöchiometriezahl von 1 entstehen nur noch Dimethylether und Kohlendioxid. Zwischen einer Stöchiometriezahl von 1 und 2 liegt eine Mischung aus Dimethylether, Wasser und Kohlendioxid vor. Durch das Reaktionsgleichgewicht sind aber immer alle Komponenten am Reaktoraustritt vorhanden. Im Fall der Bildung nennenswerter Mengen von Kohlendioxid wird Kohlendioxid in der Regel mittels einer chemischen Wäsche abgetrennt und zur Synthesegaserzeugung rezykliert oder verworfen.

Zum Begriff der Stöchiometriezahl sei auf Fachliteratur und insbesondere den eingangs zitierten Artikel von Kiendl et al. verwiesen.

Die bei einer Stöchiometriezahl von 2 und mehr bei der Dimethylethersynthese ablaufenden (Netto-)Reaktionen sind dabei die folgenden: 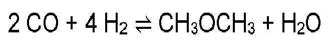 Δ H₂₉₈ = -205 kJ/mol 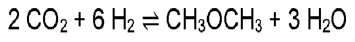 Δ H₂₉₈ = -123 kJ/mol

Bei einer Stöchiometriezahl von 1 läuft die folgende (Netto-)Reaktion ab: 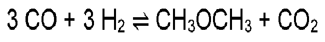 Δ H₂₉₈ = -246 kJ/mol

Im Falle eines Synthesegaseinsatzes mit einer Stöchiometriezahl von weniger als 2 muss typischerweise Kohlendioxid aus dem System entfernt werden. Eine Möglichkeit dazu besteht durch einen Abzug von Kohlendioxid aus einer zur Bearbeitung des Produktgemischs der Dimethylethersynthese eingesetzten Trennkolonne, wie beispielsweise in der EP 3 521 267 A1 beschrieben.

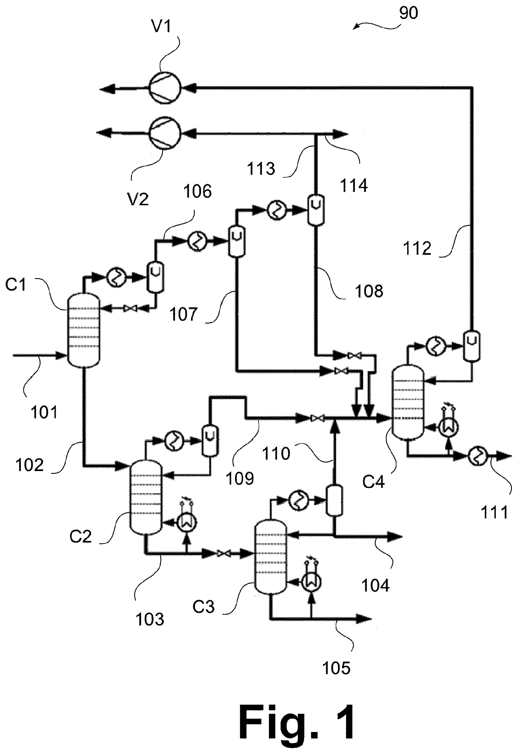

Ein hierzu verwendbares Trennverfahren, das in Figur 1 dargestellt ist und unten näher erläutert wird, ist vergleichsweise aufwendig und setzt auf vier bis fünf Trennkolonnen, nämlich eine Rückwaschkolonne (erste Trennkolonne), um Methanol und Wasser zu kondensieren, eine bei hohem Druck betrieben Trennkolonne (zweite Trennkolonne), um gelöste leichte Gase aus dem gebildeten Methanol-Wasser-Gemisch abzutrennen, eine Trennkolonne zur Trennung von Methanol und Wasser (dritte Trennkolonne), um ersteres zur Dimethylethersynthese rezyklieren und letzteres aus dem Verfahren ausschleusen zu können, die bereits erwähnte Trennkolonne zur Trennung von Dimethylether und Kohlendioxid (vierte Trennkolonne), aus deren Sumpf Dimethylether, beispielsweise mit Treibstoffspezifikation, abgezogen werden kann und an deren Kopf größtenteils Kohlendioxid mit zuvor gelösten Gasen abgezogen werden kann, und eine optionale weitere Trennkolonne (fünfte Trennkolonne) für eine Rückwäsche des zur Dimethylethersynthese zu rezyklierenden, nicht umgesetzten Synthesegases mit Dimethylether, um aus diesem mehr Kohlendioxid zu entfernen und die Konzentration von Kohlendioxid in der Dimethylethersynthese zu reduzieren.

Bei einer Trennkolonne handelt es sich im hier verwendeten Sprachgebrauch um eine Trenneinheit, die dafür eingerichtet ist, ein gasförmig oder flüssig oder in Form eines Zweiphasengemischs mit flüssigen und gasförmigen Anteilen, ggf. auch im überkritischen Zustand, eingespeistes Stoffgemisch durch ein Trennverfahren, insbesondere Rektifikation (im allgemeinen Sprachgebrauch auch als Destillation bezeichnet) zumindest teilweise aufzutrennen, also aus dem Stoffgemisch jeweils Reinstoffe oder zumindest Stoffgemische mit anderer Zusammensetzung zu erzeugen. Typischerweise sind Trennkolonnen als zylindrische Metallbehälter ausgebildet, die mit Einbauten, beispielsweise Trennböden oder geordneten oder ungeordneten Packungen, in einem Trennbereich ausgerüstet sind.

Eine Trennkolonne zur klassischen Rektifikation weist einen Sumpfverdampfer bzw. Sumpfaufkocher auf. Hierbei handelt es sich um eine Einrichtung mit einem Wärmeübertrager bzw. Heizer, der dafür eingerichtet ist, eine im Sumpf der Trennkolonne, d.h. einem Bereich unterhalb des Trennbereichs, anfallende flüssige Fraktion, auch als Sumpfflüssigkeit oder Sumpfprodukt bezeichnet, zu erwärmen. Mittels eines Sumpfverdampfers wird kontinuierlich ein Teil des Sumpfprodukts verdampft und gasförmig in den Trennbereich zurückgespeist. Ein anderer Teil des Sumpfprodukts wird typischerweise unverdampft aus der Trennkolonne abgezogen.

Trennkolonnen weisen ferner typischerweise einen Kopfkondensator auf. Hierbei handelt es sich um eine Einrichtung mit einem Wärmeübertrager zur Kühlung, der dafür eingerichtet ist, eine am Kopf der Trennkolonne, d.h. einem Bereich oberhalb des Trennbereichs, anfallende gasförmige Fraktion, auch als Kopfgas oder Kopfprodukt bezeichnet, unter Erhalt eines Kondensats zumindest teilweise zu kondensieren. Mittels eines Kopfkondensators wird kontinuierlich ein Teil des Kopfprodukts kondensiert und flüssig als Rücklauf auf den Trennbereich aufgegeben. Nicht entsprechend rückgeführtes Kondensat, alternativ oder zusätzlich aber auch ein im Kopfkondensator nicht kondensierender Anteil des Kopfprodukts, oder ein Teil hiervon, kann flüssig oder gasförmig abgezogen werden.

Stoffströme, Gasgemische, Kopfprodukte, Sumpfprodukte usw. können im hier verwendeten Sprachgebrauch reich oder arm an einer oder mehreren Komponenten sein, wobei die Angabe "reich" für einen Gehalt von wenigstens 99%, 99,5%, 99,9% oder 99,99% und die Angabe "arm" für einen Gehalt von höchstens 1%, 0,5%, 0,1% oder 0,01% auf molarer, Gewichts- oder Volumenbasis stehen kann. Sind mehrere Komponenten angegeben, kann sich die Angabe "reich" oder "arm" auf die Summe aller Komponenten beziehen. Ist hier von "Methanol" oder einem "Methanol-Wasser-Gemisch" die Rede, kann es sich um einen Reinstoff, aber auch um ein an der einen bzw. den mehreren genannten Komponente(n) reiches Gemisch handeln.

Stoffströme, Gasgemische usw. können im hier verwendeten Sprachgebrauch außerdem "angereichert" oder "abgereichert" an einer oder mehreren Komponenten sein, wobei sich diese Begriffe auf einen Gehalt in einem Ausgangsgemisch beziehen. Sie sind "angereichert", wenn sie zumindest den 1,5-fachen, 2-fachen, 5-fachen, 10-fachen, 100-fachen oder 1.000-fachen Gehalt, "abgereichert", wenn sie höchstens den 0,75-fachen, 0,5-fachen, 0,1-fachen, 0,01-fachen oder 0,001-fachen Gehalt einer oder mehrerer Komponenten, bezogen auf das Ausgangsgemisch, enthalten.

Die zuvor erläuterte Trennkolonnenkonfiguration ist recht aufwendig. Gemäß Aspekten der vorliegenden Erfindung, die sich vor allem für kleine und mittlere Anlagenkapazitäten eignet, wird daher eine vereinfachte Konfiguration vorgeschlagen, die insbesondere Investitionskosten einspart.

Zur Vereinfachung des Verständnisses der vorliegenden Erfindung wird wiederum auf die Figuren verwiesen. Ein nicht erfindungsgemäßes Verfahren ist dabei, wie erwähnt, in Figur 1 dargestellt. Ein Aspekt der vorliegenden Erfindung kann insbesondere als ein Weglassen der zuvor erläuterten zweiten Trennkolonne, in Figur 1 mit C2 bezeichnet, gesehen werden, in welcher in herkömmlichen Verfahren Dimethylether, Kohlendioxid und gelöste leichte Gase aus dem in der ersten Trennkolonne abgeschiedenen Gemisch mit Wasser und Methanol entfernt werden, um diese in die in einem herkömmlichen Verfahren verwendete vierte Trennkolonne (Dimethylether-Kohlendioxid-Trennkolonne, Kolonne C4 in Figur 1) einspeisen zu können.

Diese gelösten Gase fallen nun mit Methanol am Kopf einer Trennkolonne an, die funktional zumindest zum Teil der dritten Trennkolonne des herkömmlichen Verfahrens entspricht (Kolonne C3 in Figur 1), bezogen auf das vorgeschlagene Verfahren nachfolgend aber auch als zweite Trennkolonne bezeichnet wird (der besseren Vergleichbarkeit halber in den Figuren 2 und 3, die Ausgestaltungen der Erfindung veranschaulichen, aber weiterhin mit C3 bezeichnet ist).

Aus einem entsprechenden Kopfgas wird im Kopfkondensator der zweiten Trennkolonne, falls vorhanden, flüssiges Methanol abgeschieden. Der nicht kondensierende Anteil umfasst die zuvor gelösten leichten Gase, die in dem herkömmlichen Verfahren in der dortigen zweiten Trennkolonne von dem Gemisch aus Wasser und Methanol abgetrennt werden. Diese können in die Dimethylethersynthese zurückgeführt und dabei insbesondere auf einer Zwischenstufe in die Synthesegasverdichtung eingespeist werden, z.B. mit einem Druck zwischen 1,5 oder 5 bis 10 bar. Der Anteil an gelösten Gasen, wie z.B. Dimethylether und Kohlendioxid, im flüssigen Methanol, d.h. der im Kopfkondensator der zweiten Trennkolonne gebildeten flüssigen Phase, ist hierbei höher.

Um eine Anreicherung von Kohlendioxid in der Dimethylethersynthese zur vermeiden, in die dieses Methanol ebenfalls rückgeführt wird, und damit den Umsatz hochzuhalten, wird eine Ausschleusung eines Teils dieses flüssigen Methanols vorgeschlagen.

Die vorliegende Erfindung schlägt jedoch in einigen Aspekten auch weitere Ausführungen der Trennsequenz zur Herstellung von Dimethylether vor. Für eine weitere Vereinfachung kann insbesondere die erwähnte erste Kolonne, durch mindestens einen Flashschritt (d.h. Kondensation durch Abkühlung und/oder Entspannung) ersetzt werden. Als eine weitere Ausführung kann auch die zur Trennung von Wasser und Methanol eingesetzte Trennkolonne weggelassen werden. Der Flüssigkeitsstrom aus der ersten Trennkolonne bzw. einem entsprechenden Flashschritt wird in diesem Fall zum Teil zur Dimethylethersynthese zurückgeführt. Diese weiteren Ausgestaltungen sowie entsprechende Vorteile und Randbedingungen werden unten näher erläutert.

Insgesamt schlägt die vorliegende Erfindung ein Verfahren zur Herstellung von Dimethylether vor, bei dem ein die Reaktanden Kohlenmonoxid und/oder Kohlendioxid und Wasserstoff enthaltendes Synthesegas unter teilweiser Umsetzung der Reaktanden und unter Bildung von Methanol an einem oder mehreren Methanolkatalysatoren und unter Umsetzung zumindest eines Teils des Methanols an einem oder mehreren Dimethyletherkatalysatoren einer Dimethylethersynthese unterworfen wird, wobei der oder die Methanolkatalysator(en) und der oder die Dimethyletherkatalysator(en) in einem gemeinsamen Katalysatorbett bereitgestellt sind. Die vorliegende Erfindung betrifft also eine zuvor erläuterte Direktsynthese von Dimethylether. Der Einfachheit halber ist nachfolgend lediglich von einer "Dimethylethersynthese" die Rede.

Der Dimethylethersynthese wird ein Rohprodukt entnommen, das neben Dimethylether auch Wasser, Methanol und leichte Gase enthält, wobei die leichten Gase Kohlendioxid, Kohlenmonoxid und Wasserstoff umfassen. Zumindest ein Teil des Rohprodukts wird einer ersten Trennung unterworfen, in der ein gegenüber dem Rohprodukt an Wasser und Methanol angereichertes und an Dimethylether und den leichten Gasen abgereichertes, aber einen Teil der leichten Gase enthaltendes, flüssiges Trennprodukt gebildet wird. Diese erste Trennung kann, wie in bekannten Verfahren üblich und oben erläutert, insbesondere unter Verwendung der "ersten" Trennkolonne einer entsprechenden Trennsequenz durchgeführt werden, im Rahmen der vorliegenden Erfindung aber auch durch Einsatz eines oder mehrerer Flashschritte.

Gemäß den hier vorgeschlagenen Ausgestaltungen der Erfindung wird bzw. werden aus zumindest einem Teil des flüssigen Trennprodukts der ersten Trennung unter Verwendung keiner oder höchstens einer zweiten Trennung ein oder mehrere Rückführströme gebildet. Von dem einen oder den mehreren Rückführströmen, die nicht unerhebliche Mengen an Kohlendioxid enthalten, wird ein oder jeweils ein Teil aus dem Verfahren ausgeführt, um eine Anreicherung insbesondere dieses Kohlendioxids in dem gebildeten Kreislauf zu vermeiden (Purge). Ein oder jeweils ein verbleibender Rest wird zumindest zum Teil oder jeweils zumindest zum Teil zu der Dimethylethersynthese zurückgeführt.

Durch die hier vorgeschlagenen Maßnahmen können damit eine bis zu drei Trennkolonnen des bereits zuvor erläuterten herkömmlichen Trennverfahrens eingespart werden. Das vorgeschlagene Konzept mit weniger Trennkolonnen ist vor allem für kleinere und mittlere Anlagen zur Herstellung von Dimethylether geeignet.

In Ausgestaltungen der vorliegenden Erfindung wird ein das die Reaktanden Kohlenmonoxid und/oder Kohlendioxid und Wasserstoff enthaltende Synthesegas, oder zumindest ein Teilstrom oder eine Komponente hiervon, mittels eines Einsatzverdichters von einem Bereitstellungsdruck auf einen Einspeisedruck zur Dimethylethersynthese verdichtet und der eine oder zumindest einer der mehreren Rückführströme werden in den Einsatzverdichter eingespeist.

In einer Ausgestaltung der vorliegenden Erfindung kann die erste Trennung unter Verwendung einer Trennkolonne durchgeführt werden, in die zumindest ein Teil des Rohprodukts eingespeist wird, und in der das flüssige Trennprodukt als flüssiges Sumpfprodukt gebildet wird. Funktion und Ausgestaltung einer entsprechenden ersten Trennung kann im Wesentliche jener der ersten Trennkolonne in dem bekannten Verfahren entsprechen. Eine derartige Trennkolonne wird also in dieser Ausgestaltung im Wesentlichen beibehalten.

Wie erwähnt, kann die erste Trennung in alternativen Ausgestaltungen der vorliegenden Erfindung aber auch ein Abkühlen und eine Phasentrennung, beispielsweise durch Flaschen zumindest eines Teils des Rohprodukts umfassen, wodurch das flüssige Trennprodukt als flüssiges Trennprodukt dieser Phasentrennung gebildet wird. Eine Entspannung kann dabei vorgesehen sein, ist jedoch nicht erfindungswesentlich. Sie erfolgt typischerweise erst im letzten Schritt vor der Einspeisung in einen Abscheidebehälter. In der Abkühlkette wird dagegen typischerweise nicht entspannt. In derartigen Ausgestaltungen werden, insbesondere dann, wenn nur ein Flashschritt eingesetzt wird, die Abkühl- und Entspannungsbedingungen vorteilhafterweise derart gewählt, dass durch das Entspannen eine Dimethyletherfraktion erhalten wird, die das Methanol in einem zulässigen (spezifikationsgerechten) Gehalt aufweist.

In entsprechenden Ausgestaltungen kann das Abkühlen ein Kühlen unter Verwendung von Kühlluft, Kühlwasser und/oder eines weiteren Kühlmediums auf eine Temperatur von 60 bis 80 °C, insbesondere ca. 70 °C, 30 bis 50 °C, insbesondere ca. 40 °C, oder 5 bis 15 °C, insbesondere ca. 10 °C, erfolgen. Insbesondere ein Abkühlschritt auf eine Temperatur von 5 bis 15 °C, insbesondere ca. 10 °C, und eine entsprechende Entspannung ermöglicht es typischerweise ein Dimethyletherprodukt mit gängigen Spezifikationen hinsichtlich des Methanolgehalts zu erhalten. In Ausgestaltungen der Erfindung können auch mehrere entsprechender Abkühlschritte nacheinander durchgeführt werden, insbesondere mit einem oder mehreren, sich am Ende anschließenden Entspannungsschritten.

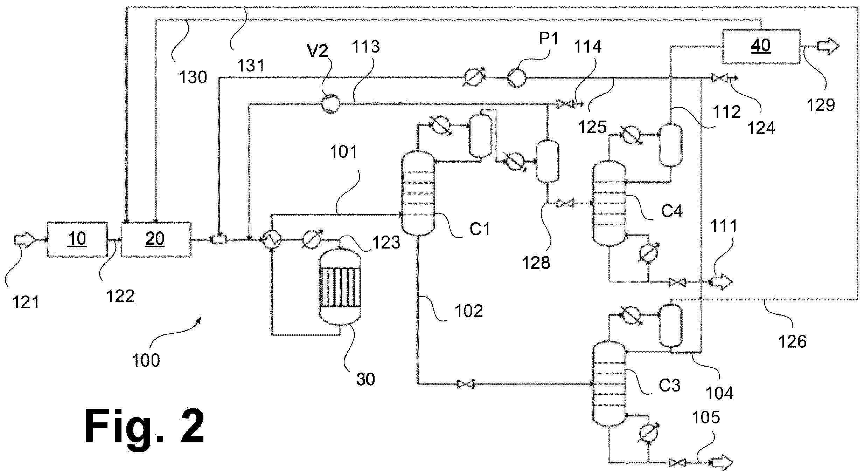

In Ausgestaltungen der vorliegenden Erfindung wird die zweite Trennung durchgeführt und es wird für die zweite Trennung eine Trennkolonne verwendet, in die zumindest ein Teil des flüssigen Trennprodukts eingespeist wird, wie in Figur 2 dargestellt. In der für die zweite Trennung verwendeten Trennkolonne werden ein flüssiges Sumpfprodukt und ein gasförmiges Kopfprodukt gebildet, und der eine oder die mehreren Rückführströme wird bzw. werden unter Verwendung des gasförmigen Kopfprodukts dieser Trennkolonne gebildet. Hierbei kann auch eine teilweise Kondensation des Kopfprodukts erfolgen, so dass die erwähnten leichten Komponenten (in einem Gasstrom) und Methanol (in einem Flüssigstrom) separat zurückgeführt werden.

Entsprechende Ausgestaltungen der vorliegenden Erfindung sind insbesondere dann besonders vorteilhaft, wenn das Rohprodukt auf einem Druck in einem Bereich von 5 bis 10 bar Absolutdruck bereitgestellt wird, wobei die Trennkolonne, die für die zweite Trennung verwendet wird, in diesem Fall auf einem Druck in diesem Druckbereich betrieben werden kann. Die Rückführung erfolgt hierbei insbesondere auf einem entsprechenden Betriebsdruck von 5 bis 10 bar Absolutdruck. Auf diese Weise lässt sich ein Verdichter einsparen. Entsprechende Bedingungen liegen insbesondere dann vor, wenn das eingesetzte Synthesegas unter Verwendung eines Vergasungsverfahrens bereitgestellt wird.

In entsprechenden Ausgestaltungen kann das in der für die zweite Trennung verwendeten Trennkolonne gebildete flüssige Sumpfprodukt als Wasserfraktion aus dem Verfahren ausgeführt werden.

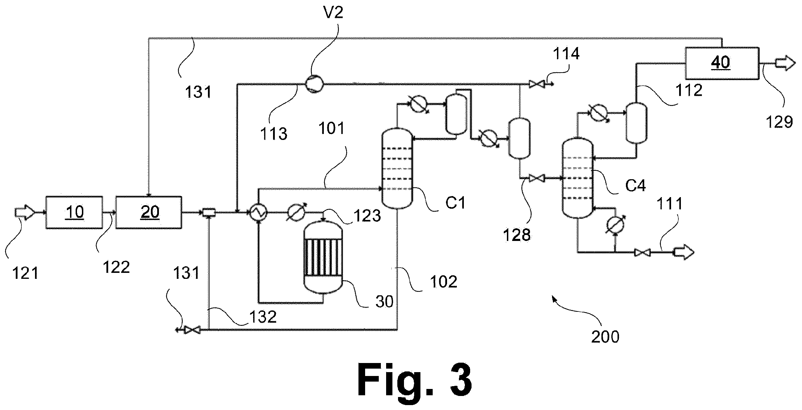

In anderen Ausgestaltungen der Erfindung kann aber auch, wie bereits erwähnt, vorgesehen sein, dass keine zweite Trennung durchgeführt wird, wobei der Rückführstrom mit derselben Zusammensetzung wie das Trennprodukt der ersten Trennung gebildet wird, wie in Figur 3 dargestellt. Mit anderen Worten wird hier auf die in herkömmlichen Verfahren vorhandene Trennkolonne zur Trennung von Wasser und Methanol verzichtet.

Eine derartige Ausgestaltung des erfindungsgemäßen Verfahrens ist insbesondere dann vorteilhaft, wenn bei der Dimethylethersynthese kein oder jedenfalls nur wenig Wasser gebildet wird. Wie eingangs erläutert, ist dies insbesondere dann der Fall, wenn das Synthesegas mit einem Verhältnis von Wasserstoff zu Kohlenmonoxid von 0,8 bis 1,2, insbesondere ca. 1,0 bereitgestellt wird. Dies ist daher in einer entsprechenden Ausgestaltung der Erfindung vorteilhafterweise vorgesehen. Andere Ausgestaltungen der Erfindung, können dagegen unter Verwendung von Synthesegasen mit höheren Verhältnissen von Wasserstoff zu Kohlenmonoxid eingesetzt werden.

Im Rahmen der vorliegenden Erfindung können grundsätzlich alle Verfahren zur Herstellung von Synthesegas eingesetzt werden, so dass das Synthesegas Komponenten umfassen kann, die durch eine Vergasung von Biomasse, Müll oder anderen Einsatzstoffen erhalten werden. In diesen Fällen wird insbesondere ein Synthesegas mit einer Stöchiometriezahl von weniger als 2 erhalten, wobei sich Verfahrensvarianten ohne zweite Trennung besonders eignen.

In anderen Ausgestaltungen der Erfindung kann unter Erhalt eines Synthesegases mit einer Stöchiometriezahl von 2 oder mehr eine Dampfreformierung und/oder Trockenreformierung, mit oder ohne Einstellung des Wasserstoffgehalts durch Zugabe von Wasserstoff und/oder eine reverse Wassergasshift, zum Einsatz kommen. Hierbei eignen sich insbesondere Verfahrensvarianten mit zweiter Trennung eine Hochtemperaturelektrolyse mit reverser Wassergasshift, bzw. Hochtemperatur-Ko-Elektrolyse ist in solchen Fällen vorteilhaft einsetzbar.

Eine Hochtemperaturelektrolyse kann insbesondere unter Verwendung einer oder mehrerer Festoxidelektrolysezellen durchgeführt werden, wie sie auch für eine Kohlendioxidelektrolyse zum Einsatz kommen kann. Für weitere Details sei auf Druckschriften wie die WO 2018/228717 A1 verwiesen. Eine Ko-Elektrolyse kann insbesondere eine gleichzeitige Elektrolyse von Wasser und Kohlendioxid in entsprechenden Anordnungen umfassen..

In sämtlichen Ausgestaltungen der vorliegenden Erfindung kann bei der ersten Trennung gasförmiges, gegenüber dem Rohprodukt an Dimethylether und den leichten Gasen angereichertes und an Wasser und Methanol abgereichertes Kopfprodukt gebildet werden, wobei aus zumindest einem Teil des Kopfprodukts ein Dimethyletherprodukt, das zumindest zum Teil aus dem Verfahren ausgeführt wird, und ein oder mehrere weitere Rückführströme, von dem oder denen zumindest ein Teil oder jeweils zumindest ein Teil zu der Dimethylethersynthese zurückgeführt wird, gebildet wird oder werden.

Eine Anlage zur Herstellung von Dimethylether, die dafür eingerichtet ist, ein die Reaktanden Kohlenmonoxid und/oder Kohlendioxid und Wasserstoff enthaltendes Synthesegas unter teilweiser Umsetzung der Reaktanden und unter Bildung von Methanol an einem oder mehreren Methanolkatalysatoren und unter Umsetzung zumindest eines Teils des Methanols an einem oder mehreren Dimethyletherkatalysatoren einer Dimethylethersynthese zu unterwerfen, wobei der eine oder die mehreren Methanolkatalysatoren und der eine oder die mehreren Dimethyletherkatalysator in einem gemeinsamen Katalysatorbett bereitgestellt sind, und wobei die Anlage ferner dafür eingerichtet ist, der Dimethylethersynthese ein Rohprodukt zu entnehmen, das neben Dimethylether auch Wasser, Methanol und leichte Gase enthält, wobei die leichte Gase Kohlendioxid, Kohlenmonoxid und Wasserstoff umfassen, zumindest einen Teil des Rohprodukts einer ersten Trennung zu unterwerfen, und in der ersten Trennung ein gegenüber dem Rohprodukt an Wasser und Methanol angereichertes und an Dimethylether und den leichten Gasen abgereichertes, aber einen Teil der leichten Gase enthaltendes, flüssiges Trennprodukt zu bilden, ist ebenfalls Gegenstand der Erfindung. Hierbei ist vorgesehen, dass die Anlage dafür eingerichtet ist, aus zumindest einem Teil des flüssigen Trennprodukts unter Verwendung keiner oder höchstens einer zweiten Trennung ein oder mehrere Rückführströme zu bilden und von dem einen oder den mehreren Rückführströmen einen oder jeweils einen Teil aus dem Verfahren auszuführen einen oder jeweils einen verbleibenden Rest hiervon zumindest zum Teil oder jeweils zumindest zum Teil zu der Dimethylethersynthese zurückzuführen.

Zu Merkmalen und Vorteilen einer entsprechenden Anlage und möglicher Ausgestaltungen sei auf die obigen Erläuterungen bezüglich des erfindungsgemäßen Verfahrens und seiner Ausgestaltungen ausdrücklich verwiesen.

Anlagen gemäß Ausgestaltungen der vorliegenden Erfindung weisen insbesondere Mittel auf, die sie zur Durchführung eines vorstehend beschriebenen Verfahrens und entsprechender Ausgestaltungen ertüchtigen. Die erfindungsgemäße Anlage bzw. deren vorteilhafte Ausgestaltungen profitieren dementsprechend von den Vorteilen des jeweils entsprechenden Verfahrens in analoger Weise und umgekehrt.

Im Folgenden werden weitere Merkmale und Vorteile der vorliegenden Erfindung anhand von Ausführungsbeispielen unter Bezugnahme auf die beiliegende Zeichnung näher erläutert.

### Kurze Beschreibung der Figuren

Figur 1 zeigt eine nicht erfindungsgemäße Ausgestaltung eines Verfahrens zur Bearbeitung eines Rohprodukts einer Dimethylethersynthese.
Figur 2 zeigt ein Verfahren zur Synthese von Dimethylether und Bearbeitung eines Rohprodukts gemäß einer Ausgestaltung der Erfindung.
Figur 3 zeigt ein Verfahren zur Synthese von Dimethylether und Bearbeitung eines Rohprodukts gemäß einer Ausgestaltung der Erfindung.

In den Figuren werden Verfahrensschritte und Vorrichtungskomponenten teilweise gemeinsam beschrieben bzw. mit identischen Bezugszeichen angegeben. Generell betreffen Erläuterungen zu Verfahrensschritten die entsprechenden Vorrichtungskomponenten in gleicher Weise und umgekehrt. Baulich oder funktionell gleiche oder vergleichbare Komponenten sind ggf. mit identischen Bezugszeichen angegeben.

### Ausführungsform(en) der Erfindung

In Figur 1 ist ein nicht erfindungsgemäßes Verfahren zur Bearbeitung eines Rohprodukts aus einer direkten Dimethylethersynthese in Form eines vereinfachten Prozessflussdiagramms veranschaulicht und insgesamt mit 90 bezeichnet. Das Verfahren umfasst im dargestellten Beispiel die Verwendung von vier Trennkolonnen C1, C2, C3 und C4 mit jeweils in üblicher Weise ausgeführten Sumpfverdampfern (nicht bei der ersten Trennkolonne C1) und Kopfkondensatoren, die mit geeigneten Heizmedien bzw. Kühlmedien betrieben werden können.

Ein nach der direkten Dimethylethersynthese vorliegendes Rohprodukt wird in Form eines Stoffstroms 101 bereitgestellt, abgekühlt, z.B. durch Wärmeintegration mit dem Reaktionseinsatzgas (nicht veranschaulicht), und in die erste Trennkolonne C1 geleitet. Das Sumpfprodukt 102 der ersten Trennkolonne, im Wesentlichen eine Mischung aus Wasser und Methanol mit darin gelöstem Dimethylether und gelösten Gasen, wird in der zweiten Trennkolonne C2 von Dimethylether und den gelösten Gasen befreit, so dass in der zweiten Trennkolonne C2 ein (überwiegend) Methanol und Wasser enthaltendes Sumpfprodukt 103 entsteht. Das Methanol wird in der dritten Trennkolonne C3 abgetrennt und beispielsweise in Form eines Stoffstroms 104 zur Dimethylethersynthese rezykliert. Wasser wird beispielsweise in Form eines Stoffstroms 105 aus dem System bzw. dem hier dargestellten Verfahren 90 entfernt.

Ein Kopfstrom 106 aus der ersten Trennkolonne C1 wird stufenweise abgekühlt, wodurch an Dimethylether reiche Kondensatströme 107, 108 gebildet werden. Diese werden in die vierte Trennkolonne (Dimethylether-Kohlendioxid-Trennkolonne) C4 eingeleitet. In der vierten Trennkolonne, in die auch ein Kopfgasstrom 109 aus der zweiten Trennkolonne C2 und nicht kondensiertes Kopfgas der dritten Trennkolonne in Form eines Stoffstroms 110 eingespeist wird, wird als Sumpfprodukt Dimethylether, z.B. mit Kraftstoffspezifikation, erhalten und als Produktstrom 111 abgezogen. Am Kopf der vierten Trennkolonne C4 befinden sich Kohlendioxid und leichtere gelöste Gase wie Wasserstoff und Kohlenmonoxid. Ein aus - unter den vorliegenden Bedingungen - nicht kondensierenden Anteilen desselben gebildeter Stoffstrom 112 kann über einen Verdichter V1 zur Dimethylethersynthese rezykliert werden. Ebendies gilt für nicht kondensierte Anteile des Kopfstroms 106 der ersten Trennkolonne C1, hier in Form eines Stoffstroms 113 dargestellt. Ein hiervon nicht über einen Verdichter V2 zurückgeführter Anteil kann zur Vermeidung der Anreicherung inerter Komponenten aus dem Verfahren ausgeleitet werden, wie mit einem Stoffstrom 114 veranschaulicht. Entsprechendes ist auch für Stoffstrom 112 möglich.

Um weiteres Kohlendioxid aus dem Gasrecycle zur Dimethylethersynthese, insbesondere in dem Stoffstrom 112, zu entfernen, kann ein entsprechender Stoffstrom mit Dimethylether gewaschen werden. Dies kann insbesondere bei den eingangs genannten Randbedingungen vorteilhaft sein, um den Kohlendioxidgehalt in der Dimethylethersynthese niedrig und den Umsatz hochzuhalten.

Figur 2 zeigt, insgesamt mit 100 bezeichnet, ein Verfahren zur Synthese von Dimethylether und Bearbeitung eines Rohprodukts gemäß einer Ausgestaltung der Erfindung in Form eines vereinfachten Prozessflussdiagramms.

Wie bereits zuvor erläutert, kann ein Aspekt der vorliegenden Erfindung, der auch in dem Verfahren 100 realisiert ist, insbesondere als ein Weglassen der zuvor erläuterten zweiten Trennkolonne C2 gesehen werden, in welcher in herkömmlichen Verfahren Dimethylether, Kohlendioxid und gelöste Gase aus dem in der ersten Trennkolonne C1 abgeschiedenen Gemisch mit Wasser und Methanol entfernt werden, um diese in die vierte Trennkolonne (Dimethylether-Kohlendioxid-Trennkolonne) C4 einspeisen zu können. Die Trennkolonnen sind, soweit vorhanden, daher in Figur 2 mit denselben Bezugszeichen wie in Figur 1 veranschaulicht. Ebendies gilt, soweit vorhanden, für die einander entsprechenden Stoffströme.

Ferner sind in Figur 2 ein Synthesegaserzeugungsschritt 10, ein Verdichtungsschritt 20, ein Dimethylethersyntheseschritt 30 und ein Kohlendioxidaufreinigungsschritt 40 dargestellt, die Teil des Verfahrens 100 sind.

Dem Verfahren 100 wird beispielsweise ein kohlenwasserstoffhaltiger Einsatz mit Dampf in einem Stoffstrom 121 zugeführt und in dem Synthesegaserzeugungsschritt 10 zu Synthesegas 122 umgesetzt. Das Synthesegas 122 wird in dem Verdichtungsschritt 20, insbesondere mehrstufig, verdichtet. In den Verdichtungsschritt 20 können dabei auf Zwischenstufen rückgeführte Stoffströme eingespeist werden, wie unten erläutert. Ein unter Verwendung des Synthesegases und weiterer, unten erläuterter rückgeführter Stoffströme gebildeter Einsatzstrom 123 wird nach Erwärmung, u.a. gegen das Rohprodukt 101 der Dimethylethersynthese 30, der Dimethylethersynthese 30 zugeführt.

Zumindest ein Teil des Rohprodukts 101, das neben Dimethylether auch Wasser, Methanol, Dimethylether und gelöste Gase, darunter Kohlendioxid, Kohlenmonoxid und Wasserstoff, enthält, wird, wie zuvor, in die erste Trennkolonne C1 eingespeist, in der ein gegenüber dem Rohprodukt 101 an Wasser und Methanol angereichertes und an Dimethylether und den gelösten Gasen abgereichertes, aber zumindest einen Teil der gelösten Gase enthaltendes, flüssiges Sumpfprodukt gebildet wird. In der ersten Trennkolonne wird ferner, wie zuvor, ein gegenüber dem Rohprodukt 101 an Wasser und Methanol abgereichertes und an Dimethylether sowie den gelösten Gasen angereichertes Kopfprodukt gebildet.

Zumindest ein Teil der Sumpfflüssigkeit der ersten Trennkolonne C1 wird nun direkt in eine weitere Trennkolonne C3 überführt, die der dritten Trennkolonne C3 des Verfahrens 90 gemäß Figur 1 entspricht, nunmehr jedoch als zweite Trennkolonne bezeichnet wird. In der zweiten Trennkolonne C3 wird ein gegenüber dem Sumpfprodukt der ersten Trennkolonne an Wasser angereichertes und an Methanol sowie den gelösten Gasen abgereichertes flüssiges Sumpfprodukt gebildet, das zumindest teilweise, wie zuvor der Stoffstrom 105, als Abwasser aus dem Verfahren 100 ausgeschleust werden kann. Ferner wird in der zweiten Trennkolonne C2 ein gegenüber dem Sumpfprodukt der ersten Trennkolonne an Wasser abgereichertes und an Methanol sowie den gelösten Gasen angereichertes gasförmiges Kopfprodukt gebildet, das wie nachfolgend erläutert zur Bildung von mehreren Rückführströmen verwendet wird.

Das Kopfprodukt der zweiten Trennkolonne C3 wird dabei in der Ausgestaltung gemäß Figur 2 unter Erhalt einer Flüssigphase und einer Gasphase (hier als erste Flüssigphase und erste Gasphase bezeichnet) einer (ersten) Teilkondensation in einem Kopfkondensator der zweiten Trennkolonne C2 unterworfen. Die erste Flüssigphase ist hierbei gegenüber dem Kopfprodukt der zweiten Trennkolonne C3 an Methanol angereichert und an den gelösten Gasen abgereichert. Die erste Gasphase ist dagegen gegenüber dem Kopfprodukt der zweiten Trennkolonne C3 an Methanol abgereichert und an den gelösten Gasen angereichert. Bei der ersten Flüssigphase handelt es sich insbesondere um einen Methanolstrom 104, der, wie zuvor der Methanolstrom 104 in dem Verfahren 90, zumindest teilweise in die Dimethylethersynthese 30 zurückgeführt wird.

Da aufgrund der gegenüber dem Verfahren 90 abweichenden Verfahrensführung der Methanolstrom 104 deutlich größere Mengen an den gelösten Gasen, insbesondere an Kohlendioxid, enthält, erfolgt in dem Verfahren 100 eine Ausschleusung (Purge) eines Teils dieses Methanolstroms 104, wie hier mit 124 veranschaulicht. Ein verbleibender Anteil 125 kann mittels einer Pumpe P1 druckerhöht und/oder stromauf der Dimethylethersynthese 30 zurückbefördert werden.

Die bei der ersten Teilkondensation des gasförmigen Kopfprodukts der zweiten Trennkolonne C3 verbleibende erste Gasphase kann zumindest zum Teil in Form eines Stoffstroms 126 in die Verdichtung 20 am Eintritt oder auf einer geeigneten Zwischenstufe zurückgeführt werden.

Zumindest ein Teil des gasförmigen Kopfprodukts der ersten Trennkolonne C1 wird ebenfalls unter Erhalt einer Gasphase und einer Flüssigphase (die hier als zweite Gasphase und Flüssigphase bezeichnet werden) einer (zweiten) Teilkondensation unterworfen. Die zweite Teilkondensation kann insbesondere mehrere Kondensationsschritte umfassen und ist lediglich aus Gründen der Anschaulichkeit in Figur 2 vereinfacht dargestellt. Sie kann dem in Figur 1 veranschaulichten Verfahren 90 entsprechen. Der Strom 128 gemäß Figur 2 kann also den Strömen 107 und 108 in Figur 1 entsprechen. Zumindest ein Teil einer oder mehrerer zweiter Flüssigphasen, die aus dem Kopfgas der ersten Trennkolonne C1 gebildet wird, wird in Form eines Stoffstroms 128 (der, wie soeben erläutert, für mehrere Stoffströme stehen kann) in eine weitere Trennkolonne C4 überführt, die der vierten Trennkolonne C4 des Verfahrens 90 gemäß Figur 1 entspricht, nunmehr jedoch als dritte Trennkolonne bezeichnet wird.

Die bei der zweiten Teilkondensation des gasförmigen Kopfprodukts der ersten Trennkolonne C1 verbleibende zweite Gasphase kann zumindest zum Teil in Form eines dem Stoffstrom 113 des Verfahrens 90 entsprechenden Stoffstroms 113 wie zuvor erläutert zurückgeführt und in dem Verdichter V2 verdichtet werden. Ein Purgestrom 114 kann ebenfalls in entsprechender Weise gebildet werden.

Ein flüssiges Sumpfprodukt der dritten Trennkolonne C4 kann, wie Stoffstrom 111 in dem Verfahren 90, als Dimethyletherprodukt ausgeleitet werden. Wie Stoffstrom 112 gemäß Verfahren 90 kann ein entsprechender Stoffstrom 112 auch hier gebildet werden. Dieser wird jedoch hier durch die Kohlendioxidaufreinigung 40 geführt, in der ein Kohlendioxidstrom 129 abgetrennt wird. Der verbleibende Rest kann in Form des Stoffstroms 130 zumindest zum Teil in das Verfahren 100 zurückgeführt werden, genauer auf eine Zwischenstufe des Verdichtungsschritts.

Figur 3 zeigt, insgesamt mit 200 bezeichnet, ein Verfahren zur Synthese von Dimethylether und Bearbeitung eines Rohprodukts gemäß einer Ausgestaltung der Erfindung in Form eines vereinfachten Prozessflussdiagramms.

Gegenüber dem in Figur 2 veranschaulichten Verfahren 100 ist in dem Verfahren 200 auch die zuvor mit C3 bezeichnete Trennkolonne weggelassen. Mit dem Stoffstrom 102 wird ein Teil der Sumpfflüssigkeit der ersten Trennkolonne C1 direkt in die Dimethylethersynthese zurückgeführt. Hierbei wird ein Purge in Form eines Stoffstroms 131 vorgenommen, um eine Anreicherung von Kohlendioxid zu vermeiden und den Umsatz hochzuhalten. Diese Anordnung ist insbesondere möglich bei Frischeinsatzzusammensetzung mit einem Verhältnis von Wasserstoff zu Kohlendioxid bei 1, weil dann kein Wasser bei der Synthese von Dimethylether aus Wasserstoff und Kohlenmonoxid gebildet wird.

## Patentansprüche

1. Verfahren (100, 200) zur Herstellung von Dimethylether, bei dem ein die Reaktanden Kohlenmonoxid und/oder Kohlendioxid und Wasserstoff enthaltendes Synthesegas unter teilweiser Umsetzung der Reaktanden und unter Bildung von Methanol an einem oder mehreren Methanolkatalysatoren und unter Umsetzung zumindest eines Teils des Methanols an einem oder mehreren Dimethyletherkatalysatoren einer Dimethylethersynthese (30) unterworfen wird, wobei der eine oder die mehreren Methanolkatalysatoren und der eine oder die mehreren Dimethyletherkatalysatoren in einem gemeinsamen Katalysatorbett bereitgestellt sind, wobei der Dimethylethersynthese (30) ein Rohprodukt (101) entnommen wird, das neben Dimethylether auch Wasser, Methanol und leichte Gase enthält, wobei die leichten Gase Kohlendioxid, Kohlenmonoxid und Wasserstoff umfassen, und wobei zumindest ein Teil des Rohprodukts (101) einer ersten Trennung unterworfen wird, in der ein gegenüber dem Rohprodukt (101) an Wasser und Methanol angereichertes und an Dimethylether und den leichten Gasen abgereichertes, aber einen Teil der leichten Gase enthaltendes, flüssiges Trennprodukt gebildet wird, **dadurch gekennzeichnet, dass** aus zumindest einem Teil des flüssigen Trennprodukts der ersten Trennung unter Verwendung keiner oder höchstens einer zweiten Trennung ein oder mehrere Rückführströme (102, 104, 126) gebildet wird oder werden, dass von dem einen oder den mehreren Rückführströmen ein oder jeweils ein Teil (124, 131) aus dem Verfahren ausgeführt wird, und dass ein oder jeweils ein verbleibender Rest hiervon zumindest zum Teil oder jeweils zumindest zum Teil zu der Dimethylethersynthese (30) zurückgeführt wird.

2. Verfahren (100, 200) nach Anspruch 1, bei dem das die Reaktanden Kohlenmonoxid und/oder Kohlendioxid und Wasserstoff enthaltende Synthesegas, oder zumindest ein Teilstrom oder eine Komponente hiervon, mittels eines Einsatzverdichters (20) von einem Bereitstellungsdruck auf einen Einspeisedruck zur Dimethylethersynthese (30) verdichtet wird, wobei der eine oder zumindest einer der mehreren Rückführströme (102, 104, 126) in den Einsatzverdichter (20) eingespeist wird.

3. Verfahren (100, 200) nach Anspruch 1 oder 2, bei dem die erste Trennung unter Verwendung einer Trennkolonne (C1) durchgeführt wird, in die zumindest ein Teil des Rohprodukts (101) eingespeist wird, und in der das flüssige Trennprodukt als flüssiges Sumpfprodukt gebildet wird.

4. Verfahren nach Anspruch 1 oder 2, bei dem die erste Trennung ein Abkühlen und eine Phasentrennung zumindest eines Teils des Rohprodukts (101) umfasst, wobei das flüssige Trennprodukt bei der Phasentrennung als flüssiges Phasentrennprodukt gebildet wird.

5. Verfahren nach Anspruch 3, bei dem das Abkühlen ein Kühlen unter Verwendung von Kühlluft, Kühlwasser und/oder eines weiteren Kühlmediums auf eine Temperatur von 60 bis 80 °C, 30 bis 50 °C oder 5 bis 15 °C umfasst.

6. Verfahren (100) nach einem der vorstehenden Ansprüche, bei dem die zweite Trennung durchgeführt wird und für die zweite Trennung eine Trennkolonne (C3) verwendet wird, in die zumindest ein Teil des flüssigen Trennprodukts der ersten Trennung eingespeist wird, wobei in der für die zweite Trennung verwendeten Trennkolonne (C3) ein gasförmiges Kopfprodukt gebildet wird, und wobei der eine oder die mehreren Rückführströme (102, 104, 126) unter Verwendung des gasförmigen Kopfprodukts dieser Trennkolonne (C3) gebildet wird oder werden.

7. Verfahren (100) nach Anspruch 5, bei dem die für die zweite Trennung verwendete Trennkolonne (C3) auf einem Druck in einem Bereich von 1,5 oder 5 bis 10 bar Absolutdruck betrieben wird, wobei der eine oder die mehreren Rückführströme (102, 104, 126), der oder die unter Verwendung des gasförmigen Kopfprodukts dieser Trennkolonne (C3) gebildet wird oder werden, auf einem Druck in diesem Druckbereich bereitgestellt wird oder werden.

8. Verfahren (200) nach einem der Ansprüche 1 bis 5, bei dem keine zweite Trennung durchgeführt wird, wobei der Rückführstrom (102) mit derselben Zusammensetzung wie das Trennprodukt gebildet wird.

9. Verfahren (200) nach Anspruch 8, bei dem das Synthesegas mit einem Verhältnis von Wasserstoff zu Kohlenmonoxid von 0,8 bis 1,2 bereitgestellt wird.

10. Verfahren (200) nach einem der Ansprüche 8 oder 9, bei dem das Synthesegas Komponenten umfasst, die durch eine Vergasung von Biomasse, Müll oder anderen Einsatzstoffen bereitgestellt werden.

11. Verfahren (100) nach einem der Ansprüche 2 bis 6, bei dem das Synthesegas Komponenten umfasst, die durch eine Hochtemperaturelektrolyse mit reverser Wassergasshift, Hochtemperatur-Ko-Elektrolyse und/oder durch Dampfreformierung und/oder Trockenreformierung bereitgestellt werden.

12. Verfahren (100, 200), bei dem bei der ersten Trennung ein gasförmiges, gegenüber dem Rohprodukt an Dimethylether und den leichten Gasen angereichertes und an Wasser und Methanol abgereichertes Kopfprodukt gebildet wird, aus zumindest einem Teil des Kopfprodukts ein Dimethyletherprodukt (111), das zumindest zum Teil aus dem Verfahren (100, 200) ausgeführt wird, und ein oder mehrere weitere Rückführströme (113, 130), von dem oder denen zumindest ein Teil oder jeweils zumindest ein Teil zu der Dimethylethersynthese (30) zurückgeführt wird, gebildet wird oder werden.

13. Anlage zur Herstellung von Dimethylether, die dafür eingerichtet ist, ein die Reaktanden Kohlenmonoxid und/oder Kohlendioxid und Wasserstoff enthaltendes Synthesegas unter teilweiser Umsetzung der Reaktanden und unter Bildung von Methanol an einem oder mehreren Methanolkatalysatoren und unter Umsetzung zumindest eines Teils des Methanols an einem oder mehreren Dimethyletherkatalysatoren einer Dimethylethersynthese (30) zu unterwerfen, wobei der eine oder die mehreren Methanolkatalysatoren und der eine oder die mehreren Dimethyletherkatalysatoren in einem gemeinsamen Katalysatorbett bereitgestellt sind, und wobei die Anlage ferner dafür eingerichtet ist, der Dimethylethersynthese (30) ein Rohprodukt (101) zu entnehmen, das neben Dimethylether auch Wasser, Methanol und leichte Gase enthält, wobei die leichten Gase Kohlendioxid, Kohlenmonoxid und Wasserstoff umfassen, zumindest einen Teil des Rohprodukts (101) einer ersten Trennung zu unterwerfen, und in der ersten Trennung ein gegenüber dem Rohprodukt (101) an Wasser und Methanol angereichertes und an Dimethylether und den leichten Gasen abgereichertes, aber einen Teil der leichten Gase enthaltendes, flüssiges Trennprodukt zu bilden, **dadurch gekennzeichnet, dass** die Anlage dafür eingerichtet ist, aus zumindest einem Teil des flüssigen Trennprodukts unter Verwendung keiner oder höchstens einer zweiten Trennung ein oder mehrere Rückführströme (102, 104, 126) zu bilden und von dem einen oder den mehreren Rückführströmen einen oder jeweils einen Teil (124, 131) aus dem Verfahren auszuführen einen oder jeweils einen verbleibenden Rest hiervon zumindest zum Teil oder jeweils zumindest zum Teil zu der Dimethylethersynthese (30) zurückzuführen.

14. Anlage nach Anspruch 13, die zur Durchführung eines Verfahrens nach einem der Ansprüche 1 bis 12 eingerichtet ist.
